**Europäisches Patentamt**

**European Patent Office**

**Office européen des brevets**

(19)

(11) Veröffentlichungsnummer: **0 242 546**
**B1**

(12) # EUROPÄISCHE PATENTSCHRIFT

(45) Veröffentlichungstag der Patentschrift:
**16.08.90**

(51) Int. Cl.⁵: **A61N 1/22**, A61N 1/32,
A61N 1/04

(21) Anmeldenummer: **87103178.7**

(22) Anmeldetag: **06.03.87**

(54) **Elektrode zum Zuführen oder Abnehmen von Signalen.**

(30) Priorität: **19.03.86 DE 3609284**

(43) Veröffentlichungstag der Anmeldung:
**28.10.87 Patentblatt 87/44**

(45) Bekanntmachung des Hinweises auf die Patenterteilung:
**16.08.90 Patentblatt 90/33**

(84) Benannte Vertragsstaaten:
**AT CH DE GB LI NL**

(56) Entgegenhaltungen:
**DE-C- 938 976**
**FR-A- 318 147**
**FR-A- 762 037**
**FR-A- 2 257 312**
**US-A- 2 943 627**

(73) Patentinhaber: **Siemens Aktiengesellschaft,
Wittelsbacherplatz 2, D-8000 München 2(DE)**

(72) Erfinder: **Helmreich, Klaus, Saalestrasse 15,
D-8520 Erlangen(DE)**

## Beschreibung

Die Erfindung bezieht sich auf eine Elektrode zum Zuführen oder Abnehmen von Signalen gemäß Oberbegriff des Patentanspruchs 1.

Bekannte Elektroden dieser Art, insbesondere Reizstromelektroden für ein- oder mehrkreisige Reizstromtherapie (z.B. Siemens-Prospekt E 510, Bestell-Nr. M-E 510/2056 "Zubehör für Reizstrom-Diagnostik und -Therapie), werden normalerweise mittels elastischen Haltebands am Körper appliziert. Anschließend wird das Halteband, das Einknöpflöcher aufweist, mit zu den Einknöpflöchern passenden Befestigungsknöpfen fixiert. Diese Art der Elektrodenapplikation ist unhandlich. Positionskorrekturen sind nur noch bedingt möglich.

Die US-PS 29 43 627 beschreibt eine ähnliche Elektrode mit Befestigungsknopf, die auch noch in einer auf einem Leitkissen sitzenden Tasche angeordnet werden kann, derart, daß der Befestigungsknopf der Elektrode aus einem Schlitz der Tasche ragt.

Die DE-PS 938 976, FR-PS 318 147, FR-PS 762 037 und FR-OS 22 57 312 beschreiben Elektroden, die mit henkelförmigen Ansätzen mit Öse zum Durchschieben eines Haltebandes versehen sind. Die Haltebänder werden mittels Schnallen oder Druckknöpfen, die an den Bandenden sitzen, befestigt. Eine Befestigung dieser Art ist weiterhin unhandlich, wenn z.B. zwei oder noch mehr Elektroden von lediglich einer einzigen Person appliziert werden sollen. Entweder benötigt diese Person beide Hände zum Fixieren der Elektroden am Applikationsort oder sie benötigt beide Hände zum Befestigen des Bandes. Eine Fixierung mit der einen Hand und Bandbefestigung mit der anderen Hand ist nicht möglich.

Aufgabe vorliegender Erfindung ist es, eine Elektrode der eingangs genannten Art aufzubauen, die mittels Halteband einfacher applizierbar ist als bisher.

Die Aufgabe wird erfindungsgemäß durch die kennzeichnenden Merkmale des Anspruchs 1 gelöst.

Bei der erfindungsgemäßen Elektrode kann beispielsweise eine erste Elektrode am Halteband eingeknöpft sein, während bei einer zweiten Elektrode das Halteband durch die Öse im Ansatz geschoben ist. Die Applikation der beiden Elektroden ist jetzt besonders einfach, da von einer Bedienungsperson die erste eingeknöpfte Elektrode mit der einen Hand in einer erwünschten Position am menschlichen oder tierischen Körper applizierbar ist, während die zweite Elektrode mit der anderen Hand relativ zur ersten Elektrode und entlang dem Halteband in eine erwünschte Position in gewissem Abstand von der ersten Elektrode verschiebbar ist. Nach Applikation der zweiten Elektrode kann das um den Körper geschlungene Ende des Haltebandes am Befestigungsknopf des Ansatzes der ersten Elektrode eingeknöpft werden, so daß das Halteband samt Elektroden jetzt am Körper fixiert ist.

Weitere Vorteile und Einzelheiten der Erfindung ergeben sich aus der nachfolgenden Beschreibung eines Ausführungsbeispiels anhand der Zeichnung und in Verbindung mit den weiteren Unteransprüchen.

Es zeigen:

Figur 1 eine Anordnung zweier Reizstromelektroden gemäß der vorliegenden Erfindung für zweikreisige Reizstromtherapie an einem Halteband,

Figur 2 eine erfindungsgemäße Reizstromelektrode in Frontansicht und

Figur 3 eine in einer Tasche angeordnete Elektrode gemäß der vorliegenden Erfindung, welche Tasche aus einem, eine Leitsubstanz enthaltenden oder zur Aufnahme einer Leitsubstanz ausgebildeten Material besteht.

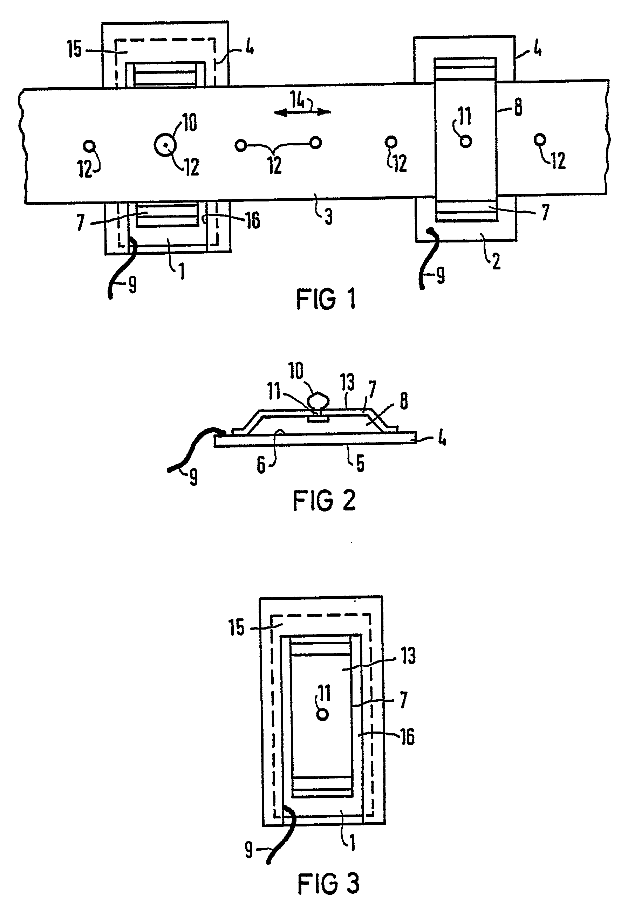

Die Figur 1 zeigt eine erste Reizstromelektrode 1 und eine zweite Reizstromelektrode 2 (beide z.B. aus dünnem Zinn oder Leitgummi), die an einem Halteband 3 zur Befestigung an einem menschlichen oder tierischen Körper angeordnet sind. Beide Reizstromelektroden 1 und 2 haben die in der Frontansicht der Figur 2 dargestellte Form. Demgemäß umfaßt jede Reizstromelektrode 1 und 2 einen flachen Elektrodenkörper 4 mit einer Applikationsfläche 5. An der der Applikationsfläche 5 abgewandten Fläche 6 des Elektrodenkörpers 4 ist ein henkelförmiger Ansatz 7 mit einer Öse 8 angebracht. Die Stromzuführungsleitung jeder Elektrode 1 und 2 ist mit der Kennziffer 9 versehen.

Gemäß den Figuren 1 und 2 bzw. auch der Figur 3 umfaßt zumindest die Reizstromelektrode 1 auch noch einen Befestigungsknopf 10, der auf dem henkelförmigen Ansatz 7 mittig in ein Einknopfloch 11 eingeknöpft ist. Mit Hilfe dieses Befestigungsknopfes kann die Elektrode 1 in eines der Einknöpflöcher 12 des Haltebandes 3 eingeknöpft werden.

Das Halteband 3 liegt also im Falle der Reizstromelektrode 1 auf der Seite 13 des henkelförmigen Ansatzes 7, die der Öse 8 abgewandt ist, auf. Bei der zweiten Reizstromelektrode 2 (die hier z.B. keinen Befestigungsknopf 10 umfaßt) ist das Befestigungsband 3 hingegen durch die Öse 8 des henkelförmigen Ansatzes 7 beweglich durchgeschoben.

Während also die erste Reizstromelektrode 1 mit einer Hand in einer gewünschten Position am menschlichen oder tierischen Körper appliziert wird, kann mit der anderen Hand die zweite Reizstromelektrode 2 entlang dem Band in Richtung des Doppelpfeiles 14 verschoben werden, bis auch sie sich in einer erwünschten Applikationsposition befindet. Anschließend kann das Halteband 3 nach Schlingenbildung durch Einknöpfen am Befestigungsknopf 10 am Ansatz 7 der ersten Elektrode 1 befestigt werden.

Reizstromelektroden der dargestellten Art werden normalerweise nicht direkt, sondern mittels leitfähiger Zwischenlage am Körper plaziert. Dadurch paßt sich die Elektrode besser an die Körperoberfläche an. Darüber hinaus ist diese Art Applikation hygienischer, da die Zwischenlage gereinigt oder ausgewechselt werden kann. Die leitfähige Zwischenlage ist z.B. ein mit Leitungswasser oder mit 1 %iger Kochsalzlösung getränkter Papier-, Schwamm- oder Stoffstreifen. Vorzugsweise ist jedoch die jeweilige Reizstromelektrode in einer Tasche 15 untergebracht, die aus einem, die Leitsubstanz aufnehmenden Material, insbesondere Stoff, besteht. Die Tasche umfaßt dabei auf der Seite des henkelförmigen Ansatzes 7 mit Öse 8 des Elektrodenkörpers 4 eine Durchtrittsöffnung 16 für den Ansatz nebst Öse. Gemäß der Figur 3 hat diese Durchtrittsöffnung 16 z.B. die Form eines Tascheneinschnittes. Zum Zwecke besserer Übersichtlichkeit ist in der Figur 1 lediglich die erste Reizstromelektrode 1 in einer Tasche 15 stekkend angedeutet. Die zweite Reizstromelektrode 2 befindet sich normalerweise ebenfalls in einer derartigen Tasche.

**Patentansprüche**

1. Elektrode zum Zuführen oder Abnehmen von Signalen am menschlichen oder tierischen Körper, insbesondere Reizstromelektrode für ein- oder mehrkreisige Reizstromtherapie, mit einem, mit einer Applikationsfläche versehenen Elektrodenkörper, der mittels Halteband applizierbar ist, wobei der Elektrodenkörper auf der der Applikationsfläche abgewandten Seite einen Ansatz mit Öse zum Durchschieben des Haltebandes aufweist, **dadurch gekennzeichnet**, daß der Ansatz (7) auf der der Öse (8) und dem Elektrodenkörper (4) abgewandten Seite (13) einen Befestigungsknopf (10) zum Einknöpfloch (12) am Halteband (3) umfaßt, wenn das Halteband nicht durch die Öse geschoben, sondern auf dem Ansatz befestigt werden soll.

2. Elektrode nach Anspruch 1, **dadurch gekennzeichnet**, daß der Ansatz (7) henkelförmig ausgebildet ist.

3. Elektrode nach Anspruch 1 oder 2, **dadurch gekennzeichnet**, daß der Befestigungsknopf (10) auf dem Ansatz (7) in ein Einknöpfloch (11) einknöpfbar ist.

4. Elektrode nach Anspruch 3, **dadurch gekennzeichnet**, daß das Einknöpfloch (11) für den Befestigungsknopf (10) auf dem Ansatz (7) mittig angeordnet ist.

5. Elektrode nach einem der Ansprüche 1 bis 4, **dadurch gekennzeichnet**, daß der Elektrodenkörper (4) in einer Tasche (15) aus einem, eine Leitsubstanz enthaltenden oder zur Aufnahme einer Leitsubstanz ausgebildeten Material anordbar ist, die auf der Seite des Ansatzes (7) mit Öse (8) des Elektrodenkörpers eine Durchtrittsöffnung (16) für den Ansatz nebst Öse umfaßt.

6. Elektrode nach Anspruch 5, **dadurch gekennzeichnet**, daß die Durchtrittsöffnung (16) die Form eines Tascheneinschnittes hat.

**Claims**

1. Electrode for supplying or receiving signals on the human or animal body, more particularly stimulus current electrode for single-circuit or multi-circuit stimulus current therapy, having an electrode body provided with an application surface and capable of being applied by means of a strap, whereby on the side directed away from the application surface the electrode body has an attachment with an eye for pushing through the strap, characterized in that on the side (13) directed away from the eye (8) and the electrode body (4) the attachment (7) comprises a fastening button (10) for buttoning the electrode body (4) into a button-hole (12) on the strap (3), if the strap is not to be pushed through the eye, but rather fastened on the attachment.

2. Electrode according to claim 1, characterized in that the attachment (7) is constructed in the shape of a handle.

3. Electrode according to claim 1 or 2, characterized in that the fastening button (10) on the attachment (7) can be buttoned into a button-hole (11).

4. Electrode according to claim 3, characterized in that the button-hole (11) for the fastening button (10) is arranged centrally on the attachment (7).

5. Electrode according to one of claims 1 to 4, characterized in that the electrode body (4) can be arranged in a pocket (15) consisting of a material containing a conducting substance or constructed to receive a conducting substance, which pocket on the side of the attachment (7) with the eye (8) of the electrode body comprises an opening (16) for the attachment in addition to the eye.

6. Electrode according to claim 5, characterized in that the opening (16) has the shape of a pocket recess.

**Revendications**

1. Electrode pour envoyer ou recevoir des signaux, placée sur le corps humain ou le corps d'un animal, notamment électrode d'application d'un courant de stimulation pour la mise en œuvre d'une thérapie à courant d'excitation à un ou plusieurs circuits, comportant un corps d'électrode, pourvu d'une surface d'application pouvant être appliquée au moyen d'une bande de maintien, le corps d'électrode possédant, sur le côté tourné à l'opposé de la surface d'application, une partie saillante comportant un œillet pour l'enfilage de la bande de maintien, caractérisée par le fait que la partie saillante (7) comporte, sur le côté (13) tourné à l'opposé de l'œillet (8) et du corps (4) de l'électrode, un bouton de fixation (10) servant à fixer le corps (4) de l'électrode dans une boutonnière (12) ménagée dans la bande de maintien (3), lorsque cette dernière ne doit pas être passée à travers l'œillet, mais être fixée sur la partie saillante.

2. Electrode suivant la revendication 1, caractérisée par le fait que la partie saillante (7) est réalisée sous la forme d'une anse.

3. Electrode suivant la revendication 1 ou 2, caractérisée par le fait que le bouton de fixation (10) peut être boutonné sur la partie saillante (7) dans une boutonnière (11).

4. Electrode suivant la revendication 3, caractérisée par le fait que la boutonnière (11) pour le bouton de fixation (10) est ménagée au centre de la partie saillante (7).

5. Electrode suivant l'une des revendications 1 à 4, caractérisée par le fait que le corps (4) de l'électrode peut être disposé dans une poche (15) réalisée en un matériau contenant une substance conductrice ou servant à loger une substance conductrice et qui comprend, sur le côté de la partie saillante (7) comportant l'œillet (8) du corps de l'électrode, une ouverture de passage (16) pour la partie saillante, en plus de l'œillet.

6. Electrode suivant la revendication 5, caractérisée par le fait que l'ouverture de passage (16) possède la forme d'une échancrure de poche.

FIG 1

FIG 2

FIG 3